# EUROPEAN PATENT APPLICATION

(11) **EP 4 289 336 A1**
(43) Date of publication of application: **13.12.2023**
(21) Application number: 22305851.2
(22) Date of filing: 10.06.2022
(51) Int. Cl.: A61B 3/028, G02C 7/02, A61B 5/16

(54) **METHOD AND SYSTEM FOR OBTAINING AN OPTICAL PRESCRIPTION BASED ON DETERMINED VALUES OF A FATIGUE PARAMETER**

(71) Applicant: Essilor International, 94220 Charenton-Le-Pont (FR)
(72) Inventor: GAO, Yi, 138946 Singapore (SG); SPIEGEL, Daniel, 424796 Singapore (SG); DROBE, Bjorn, 479251 Singapore (SG)
(74) Representative: Jacobacci Coralis Harle

(57) **Abstract**

The disclosure concerns methods for obtaining an optical prescription. The method includes determining a value of a fatigue parameter for at least two intermediate optical prescriptions among a plurality of intermediate optical prescriptions, the fatigue parameter associated with a visual fatigue level of a subject carrying out a visual task involving any kind of visual content, wherein each determined value of the fatigue parameter is associated with a respective one of the plurality of intermediate optical prescriptions; comparing the determined values of the fatigue parameter; and obtaining the optical prescription, based on the comparison of the determined values of the fatigue parameter. A system for obtaining an optical prescription is also disclosed.

## Description

### TECHNICAL FIELD

Various aspects of this disclosure relate to a method and a system for obtaining an optical prescription based on determined values of a fatigue parameter.

### BACKGROUND

Visual fatigue refers to the complex eye and vision-related problems that result from performing tasks requiring extended visual concentration or focus. Visual fatigue may be related to and used interchangeably with the terms "computer vision syndrome" and "digital eye strain" that result from prolonged near-vision work on digital devices such as computers, tablets, cell-phones and e-readers. Due to the modern lifestyle, the use of such digital devices, as well as the prevalence of visual fatigue have been increasing in the last two decades. Factors which contribute to visual fatigue include poor lighting, glare on the digital screen, improper viewing distances, long working hours, a subject's level of alertness, light spectrum, reduced contrast, reduced font size and uncorrected, under-corrected or poorly corrected refractive errors, such as uncorrected astigmatism and/or presbyopia. In certain situations, visual fatigue may not necessarily be related to the use of digital devices.

Conventional methods for obtaining an optical prescription focuses on maximizing visual acuity, and typically disregards visual fatigue levels. Further, there is usually more than one optical prescription which provides the same or similar visual acuity and an eye care practitioner has to make an arbitrary and subjective decision to pick the "right" optical prescription. It is therefore difficult for the eye care practitioner to objectively obtain an optical prescription that maximizes visual acuity and at the same time, minimizes visual fatigue. Often, a suboptimal optical prescription leads to long-term effects which manifest as increased visual fatigue, and sometimes, an optical prescription with lower visual acuity levels may in fact lead to high long-term visual comfort.

Thus, it is desired to seek improved means for determining an optical prescription which minimizes visual fatigue and discomfort, while providing good visual acuity.

### SUMMARY

It is an object of the disclosure to provide methods and a system for obtaining an optical prescription which maximizes visual acuity, and simultaneously, minimizes visual fatigue. To this end, methods and a system are provided for determining a value of a fatigue parameter for at least two intermediate optical prescriptions among a plurality of intermediate optical prescriptions; comparing the determined values of the fatigue parameter; and obtaining the optical prescription, based on the comparison of the determined values of the fatigue parameter. The methods may be implemented on a computing system, to determine the value of the fatigue parameter for the at least two intermediate optical prescriptions, and to provide the automated comparison, and obtaining of the optical prescription. The methods and system of the disclosure provides an objective means for guiding, optimizing and fine-tuning intermediate optical prescriptions by further determining visual fatigue levels, to provide an optical prescription which improves the overall eye-health of the subject. In addition, the disclosure provides a simple and quick method for obtaining an optical prescription, which may be easily implemented by an eye care practitioner.

The optical prescription may be particularly applicable as a prescription for an optical article suitable for human vision, e.g. eyewear equipment including glasses, sunglasses, contact lenses, and may be suitable for use by an emmetropic or ametropic subject.

A first aspect of the disclosure concerns a method for obtaining an optical prescription comprising: (i.) determining a value of a fatigue parameter for at least two intermediate optical prescriptions among a plurality of intermediate optical prescriptions, the fatigue parameter associated with a visual fatigue level of a subject carrying out a visual task involving any kind of visual content, wherein each determined value of the fatigue parameter is associated with a respective one of the plurality of intermediate optical prescriptions; (ii.) comparing the determined values of the fatigue parameter; and (iii.) obtaining the optical prescription, based on the comparison of the determined values of the fatigue parameter.

In various embodiments, the method further comprises determining the plurality of intermediate optical prescriptions, each intermediate optical prescription being different to another intermediate optical prescription among the plurality of intermediate optical prescriptions.

In various embodiments, the method further comprises receiving the plurality of intermediate optical prescriptions, each intermediate optical prescription being different to another intermediate optical prescription among the plurality of intermediate optical prescriptions.

In various embodiments, obtaining the optical prescription based on the comparison of the determined values of the fatigue parameter comprises (i.) selecting the determined value of the fatigue parameter corresponding to a lowest visual fatigue level, among the determined values of the fatigue parameter; (ii.) obtaining the optical prescription, based on the respective one of the intermediate optical prescriptions associated with the determined value of the fatigue parameter corresponding to the lowest visual fatigue level.

In various embodiments, each of the plurality of intermediate optical prescriptions comprises a value of a dioptric optical parameter, the dioptric optical parameter associated with a visual acuity condition of the subject.

In various embodiments, each of the plurality of intermediate optical prescriptions comprises a set of optical parameters, the set of optical parameters comprising a plurality of dioptric optical parameters and a corresponding value associated with a respective one of the plurality of dioptric optical parameters, wherein the plurality of dioptric parameters are associated with a visual acuity condition of the subject.

In various embodiments, determining the value of the fatigue parameter for each intermediate optical prescription among the plurality of intermediate optical prescriptions comprises (i.) performing at least one objective measurement relating to the subject; (ii.) performing at least one subjective measurement relating to the subject; (iii.) obtaining at least one parameter relating to information about the subject, or any combination of the above.

In various embodiments, the at least one objective measurement relating to the subject comprises: at least one pupil-tracking measurement, a critical flicker fusion frequency measurement, an accommodation micro-fluctuation measurement, a blink frequency measurement, a blink amplitude measurement, and/or a measurement relating to the subject's facial features, related to the visual fatigue level of the subject carrying out the visual task involving any kind of visual content.

In various embodiments, the at least one subjective measurement relating to the subject comprises at least one answer by the subject to at least one question, related to the visual fatigue level of the subject carrying out the visual task involving any kind of visual content.

In various embodiments, the method further comprises determining a baseline value of the fatigue parameter, the baseline value associated with a baseline visual fatigue level of the subject carrying out the visual task involving any kind of visual content, wherein the baseline value of the fatigue parameter is determined before determining the plurality of intermediate optical prescriptions.

In various embodiments, the method further comprises calculating a difference between the determined value of the fatigue parameter associated with the respective one of the plurality of intermediate optical prescription, and the baseline value of the fatigue parameter.

A second aspect of the disclosure concerns a system for obtaining an optical prescription comprising: (i.) means for determining a value of a fatigue parameter for at least two intermediate optical prescriptions among a plurality of intermediate optical prescriptions, the fatigue parameter associated with a visual fatigue level of a subject carrying out a visual task involving any kind of visual content, wherein each determined value of the fatigue parameter is associated with a respective one of the plurality of intermediate optical prescriptions; (ii.) means for comparing the determined values of the fatigue parameter; and (iii.) means for obtaining the optical prescription, based on the comparison of the determined values of the fatigue parameter.

In various embodiments, at least one of the means for determining the value of the fatigue parameter for the at least two intermediate optical prescriptions among the plurality of intermediate optical prescriptions, the means for comparing the determined values of the fatigue parameter, the means for obtaining the optical prescription, comprises a circuit.

In various embodiments, the system further comprises means for determining the plurality of intermediate optical prescriptions, each intermediate optical prescription being different to another intermediate optical prescription among the plurality of intermediate optical prescriptions, wherein the means for determining the plurality of intermediate optical prescriptions comprises the circuit.

In various embodiments, the system further comprises means for receiving the plurality of intermediate optical prescriptions, each intermediate optical prescription being different to another intermediate optical prescription among the plurality of intermediate optical prescriptions, wherein the means for receiving the plurality of intermediate optical prescriptions comprises the circuit.

Another aspect of the disclosure concerns a computer program product, comprising instructions to cause the system of the second aspect to execute the steps of the method of the first aspect.

### BRIEF DESCRIPTION OF THE DRAWINGS

The disclosure will be better understood with reference to the detailed description when considered in conjunction with the non-limiting examples and the accompanying drawings, in which:
- Figures 1A and 1B show schematic illustrations of method 100A, 100B for obtaining an optical prescription, in accordance with various embodiments;
- Figure 2 shows an exemplary embodiment 200 for obtaining the optical prescription, when the intermediate optical prescription comprises a set of optical parameters; and
- Figures 3A and 3B show schematic illustrations of exemplary embodiments 300A, 300B for obtaining the optical prescription, when the intermediate optical prescription comprises a value of a dioptric optical parameter; and
- Figure 4 shows a schematic illustration of a system 400 for obtaining an optical prescription, in accordance with various embodiments.

### DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements. As used herein, the term "and/or" includes any and all combinations of one or more of the associated listed items.

The reference signs included in parenthesis in the claims are for ease of understanding of the disclosure and have no limiting effect on the scope of the claims.

According to various embodiments, the term "optical prescription", as used herein, refers broadly to an optical measurement or optical prescription for correcting a refractive error of a subject that achieves optimal visual acuity and at the same time, minimizes visual fatigue.

According to various embodiments, the term "intermediate optical prescriptions", as used herein, may refer to intermediary prescriptions for correcting a refractive error, that may be subjectively determined by the subject during a refraction examination. During a refraction examination, there are often two of more possible subjectively determined refraction levels which achieve the same or similar visual acuity levels and immediate visual comfort. It is difficult for a subject to discriminate the fine differences in visual acuity between the determined refraction levels, and to choose an optimal optical prescription during the examination. The intermediate optical prescription may therefore refer to prescriptions, e.g. determined refraction levels, which achieve the same or similar visual acuity and immediate visual comfort for the subject. In some embodiments, the intermediate optical prescription may have value(s) that equate to or are greater than 0.02 logMAR (Logarithm of the Minimum Angle of Resolution). In an embodiment, the intermediate optical prescription includes a dioptric optical parameter. In another embodiment, the intermediate optical prescription includes a set of dioptric optical parameters. In various embodiments, the intermediate optical prescription may be a monocular measurement, e.g. in relation to one eye, or may be a binocular measurement, e.g. in relation to both eyes.

According to various embodiments, the term "value", as used herein, may refer to as a numerical value, for example, a discrete numerical value or as a range of numerical values. Alternatively, or in addition, the term "value" may refer to a text, e.g. a string of text, for example, "*yes*", or "*no*". In some embodiments, the text may be associated with, or assigned a quantitative, e.g. numerical value.

According to various embodiments, the term "subject", as used herein, may refer to any individual undergoing an optical examination, e.g. visual fatigue and/or refraction examination for obtaining the optical prescription.

According to various embodiments, the term "visual fatigue level", as used herein, refers to a level of, or a state, or an extent of visual fatigue of a subject. For example, the visual fatigue level may be associated with the occurrence of one or more visual fatigue symptoms such as but not limited to, tired eyes, blurred vision, ocular discomfort, redness, and/or concentration difficulties. In various embodiments, the visual fatigue level may refer to a subject's perception of his/her state of visual fatigue when carrying out the visual task. Alternatively, or in addition, the visual fatigue level may also include other fatigue parameters such as cognitive, general or muscular fatigue that results from the visual task.

According to various embodiments, the term "visual task", as used herein, may refer to a task assigned to a subject which includes a visual content, or any kind of visually demanding task. The visual content may be displayed on a digital device such as a screen, or may be available on paper, or available otherwise in an environment viewed by the subject. In some embodiments, the visual task may be personalized according to the subject's current optical prescription, habitual tasks, or may be adapted to the testing environment, e.g. by adjusting parameters such as font, color, contrast of the visual task.

According to various embodiments, the term "dioptric optical parameter" may refer to an objective measure of the subject's refractive error obtained during a refraction examination, and may be associated with the visual acuity condition of the subject. Non-limiting examples of the dioptric optical parameter may include a subject's spherical parameter, cylinder parameter, cylinder axis parameter, addition parameter, pupil diameter parameter, axial length parameter, binocular balance parameter. In various embodiments, the dioptric optical parameter may be monocular, or may be binocular. The dioptric optical parameter may be obtained using any refraction testing instrument, e.g. phoropter, refractor, optometer, autorefractor and/or a retinoscope.

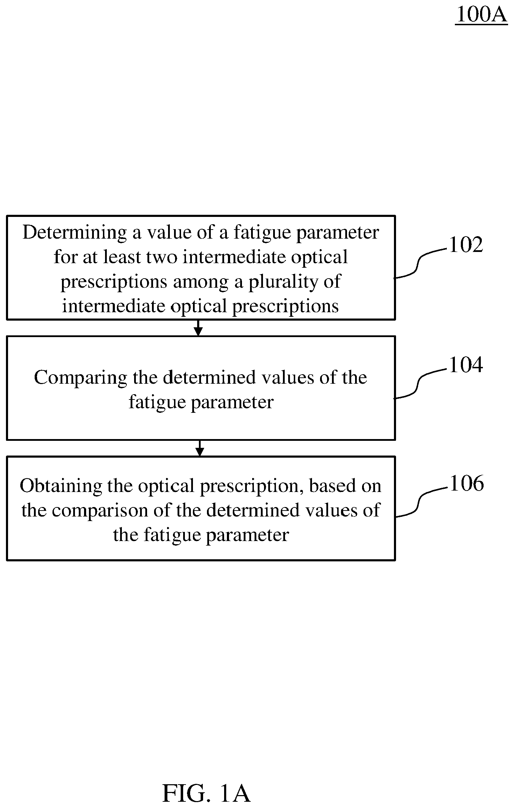

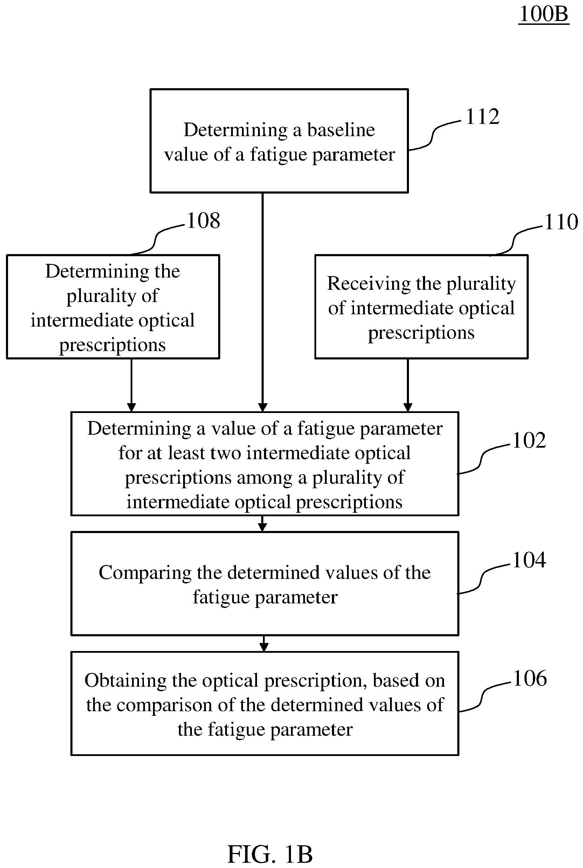

Figures 1A and 1B show schematic illustrations of method 100A, 100B for obtaining an optical prescription, in accordance with various embodiments. Referring to figure 1A, method 100A includes (i.) determining a value of a fatigue parameter ("fatigue measurement") for at least two intermediate optical prescriptions among a plurality of intermediate optical prescriptions, the fatigue parameter associated with a visual fatigue level of a subject carrying out a visual task involving any kind of visual content, wherein each determined value of the fatigue parameter is associated with a respective one of the plurality of intermediate optical prescriptions (step 102); (ii.) comparing the determined values of the fatigue parameter (step 104); and (iii.) obtaining the optical prescription, based on the comparison of the determined values of the fatigue parameter (step 106). In various embodiments, obtaining the optical prescription in step 106, may further include (i.) selecting the determined value of the fatigue parameter corresponding to a lowest visual fatigue level, among the determined values of the fatigue parameter; and (ii.) obtaining the optical prescription, based on the respective one of the intermediate optical prescriptions associated with the determined value of the fatigue parameter corresponding to the lowest visual fatigue level.

In method 100A, determining the value of the fatigue parameter, e.g. performing the fatigue measurement, for each intermediate optical prescription may include (i.) performing at least one objective measurement relating to the subject; (ii.) performing at least one subjective measurement relating to the subject; (iii.) obtaining at least one parameter relating to information about the subject, or any combination of the aforementioned steps. The at least one objective and subjective measurement may be related to, e.g. in response to, the visual fatigue level of the subject carrying out the visual task involving any kind of visual content. In some embodiments, determining the value of the fatigue parameter may include performing the at least one objective measurement, the at least one subjective measurement, and obtaining at least one parameter relating to information about the subject.

In various embodiments, the objective measurement may be made using one or more refraction testing instrument, e.g. refractors, optometers, and/or fatigue testing instrument, e.g. eye-trackers, sensors such as a camera. The fatigue testing instrument may be integrated with the refraction testing instrument, or may be a standalone device. In some embodiments, the refraction and fatigue testing instruments may cooperate with a microprocessor to determine the value of the fatigue parameter. The at least one objective measurement may include a pupil-tracking measurement, e.g. eye-tracking measurement. For example, an eye-tracker or camera may be used to track the subject's eye or pupil movements in response to the visual task in real-time, and said movements may be converted into data containing information related to pupil position, the gaze vector for each eye, and gaze point. The at least one objective measurement may also include a critical flicker fusion frequency (CFFF) measurement. For example, the transition point for an intermittent light of increasing temporal frequency, where the flickering ceases and the light is perceived as continuous by the subject may be measured. The at least one objective measurement may also include an accommodation micro-fluctuation measurement, such as but not limited to pupil micro-fluctuations and fixational eye movement parameters, which may correspond to tremors or states of temporal ocular instability due to spasms of the ciliary muscle when carrying out the visual task. The at least one objective measurement may also include a blink frequency and blink amplitude measurement. For example, an eye-tracker or camera may be used to determine the subject's blink frequency, blink amplitude, e.g. strength of eyelid closure, eyelid twitches, and/or duration of eyelid closure, in response to the visual task in real-time. The at least one objective measurement may also include a measurement relating to the subject's facial features such as the eyes, mouth or head of the subject, and the corresponding calculation of their aspect ratio. For example, a smaller eye opening, larger mouth opening, head-down frequency may be measured and may be indicative of the visual fatigue level of the subject. Any combination of the aforementioned objective measurements is possible.

In various embodiments, the at least one subjective measurement may include at least one answer by the subject to at least one question that is related to the visual fatigue level of the subject carrying out the visual task involving any kind of visual content. Thus, subjective measurements may include results relying at least partially on the subject's response, e.g. a questionnaire, which may be measured during the visual fatigue examination. Non-limiting examples of such questionnaires include symptom-based questionnaires such as the Conlon Visual Discomfort Survey, and the Convergence Insufficiency Symptoms Survey. Any combination of the aforementioned subjective measurements is possible.

In various embodiments, the at least one parameter relating to information about the subject may include personal information such as age, gender, ethnicity, country or city of residence or combinations thereof; or information related to the subject's digital device usage and behavior thereof. Any combination of the aforementioned information about the subject is possible.

It is contemplated that determining the value of a fatigue parameter for at least two intermediate optical prescriptions among a plurality of intermediate optical prescriptions may be established based on at least one predictive model which provides a relationship between the value of the fatigue parameter and the at least one objective measurement, the at least one subjective measurement, and/or the at least one parameter relating to information about the subject. The predictive model may be implemented using a machine learning algorithm, which may be selected from the group consisting of supervised, semi-supervised and unsupervised learning. For example, one or more neural networks may be trained by inputting a series of objective measurements, subjective measurements, and/or parameters relating to information about the subject, and building a correlation table or any database containing information on the relationship between the value of a fatigue parameter and the objective measurement, the subjective measurement and/or the parameter relating to information about the subject.

Referring to figure 1B, method 100B may further include determining the plurality of intermediate optical prescriptions, and each intermediate optical prescription may differ to another intermediate optical prescription among the plurality of intermediate optical prescription (step 108). The plurality of intermediate optical prescriptions may be determined during a refraction examination by a refraction testing instrument, and may include differing intermediate optical prescriptions which achieve the same or similar visual acuity levels and immediate visual comfort for the subject. In step 102, a value of a fatigue parameter is then determined for at least two of the determined intermediate optical prescriptions. In other words, the step of determining the value of the fatigue parameter may be integrated into the refraction examination process, and may be performed in relation to at least two intermediate optical prescriptions determined during the refraction examination process.

Alternatively, or in addition, method 100B may further include receiving the plurality of intermediate optical prescription, and each intermediate optical prescription may differ to another intermediate optical prescription among the plurality of intermediate optical prescription (step 110). In this embodiment, the intermediate optical prescriptions may be determined prior to determining the value of the fatigue parameter, and may be stored in an external source or the memory of a separate refraction testing instrument. In other words, the value of the fatigue parameter may be determined in a separate measurement, e.g. standalone device, and may not be integrated with the refraction examination process for determining the intermediate optical prescriptions.

In some embodiments, method 100B may include both determining the intermediate optical prescriptions (step 108), and receiving the intermediate optical prescriptions (step 110). The value of the fatigue parameter may be determined for the determined and received plurality of intermediate optical prescriptions.

As shown in figure 1B, method 100B may further include determining a baseline value of the fatigue parameter, which is associated with the baseline visual fatigue level of the subject carrying out the visual task involving any kind of visual content (step 112). The baseline value of the fatigue parameter may be determined prior to determining the plurality of intermediate optical prescriptions. For example, the at least one of the aforementioned objective and/or subjective measurement may be performed, and/or parameter relating to information about the subject may be obtained, prior to the refraction examination process for determining (step 108) and/or receiving (step 110) the plurality of intermediate optical prescriptions. In an example, the baseline value of the fatigue parameter may be determined at the beginning of the visual fatigue testing process to determine the value of a fatigue parameter for at least two intermediate optical prescriptions among the plurality of intermediate optical prescriptions (step 102).

In various embodiments, method 100B further includes calculating a difference between the determined value of the fatigue parameter associated with the respective one of the plurality of intermediate optical prescriptions (step 106), with the baseline value of the fatigue parameter (step 112). This may allow the eye care practitioner to separate the visual fatigue level associated with the respective one of the intermediate optical prescriptions, from the visual fatigue level due to the examination process itself, and from other types of fatigue, e.g. cognitive, general, muscular fatigue.

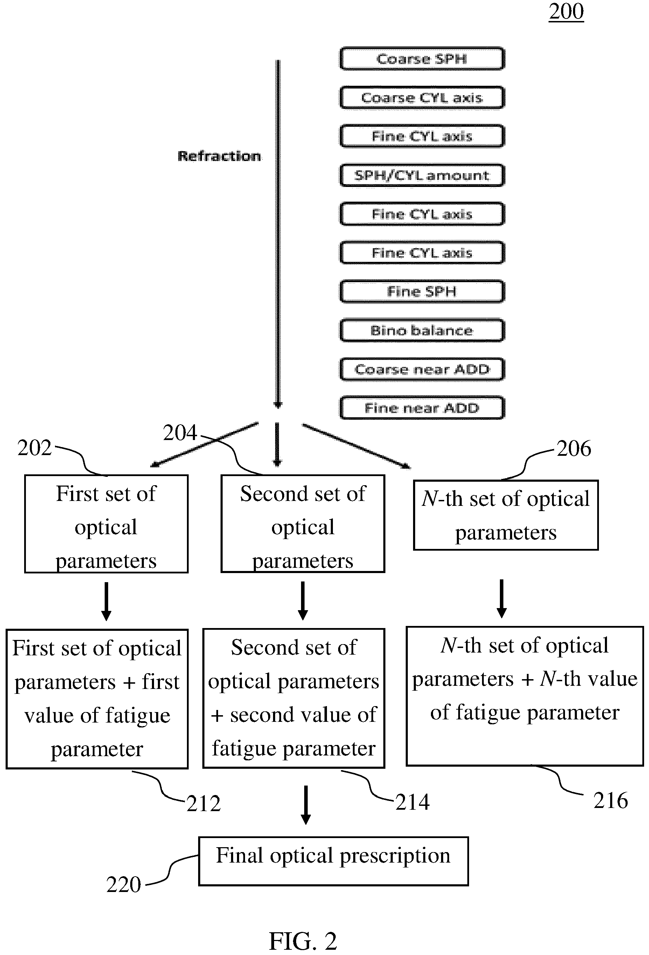

Figure 2 shows an exemplary embodiment 200 for obtaining the optical prescription, when the intermediate optical prescription comprises a set of optical parameters. In some embodiments, the set of optical parameters, e.g. intermediate optical prescriptions, may include a plurality of dioptric optical parameters, each having a corresponding value associated with a respective one of the plurality of dioptric optical parameters. The plurality of dioptric optical parameters may be associated with a visual acuity condition of the subject. For example, the set of dioptric optical parameters may include a value for a subject's spherical parameter, a value for the subject's cylinder parameter, and a value for the subject's cylinder axis parameter, which are obtained from the refraction examination. The set of dioptric optical parameters may be used to correct the subject's refractive error to provide good visual acuity.

Referring to figures 1A, 1B and 2, the plurality of intermediate optical prescriptions may be determined during the refraction examination (step 108); and/or received from an external source (step 110). As shown in figure 2, the plurality of intermediate optical prescriptions may include a first set of optical parameters 202, a second set of optical parameters 204, and up to an *N*-th, *N* ≥'2, set of optical parameters 206, each set of optical parameters providing the same or similar visual acuity for the subject.

Step 102 may thus include determining the value of the fatigue parameter, e.g. fatigue measurements, for at least two sets of the first to *N*-th sets of optical parameters 202, 204, 206. As shown in figure 2, a first value of the fatigue parameter 212 for the first set of optical parameters 202, a second value of the fatigue parameter 214 for the second set of optical parameters 204, and up to an *N*-th, *N* ≥ 2, value of the fatigue parameter 216 for the *N*-th set of optical parameters 206, may be obtained.

Step 104 may thus include comparing the determined values of the fatigue parameter. For example, the first value 212, the second value 214, and up to the *N*-th value 216 of the fatigue parameter may be compared against each other. In some embodiments, step 104 may further include calculating the difference between the determined values of the fatigue parameter, e.g. the first value 212, the second value 214, and up to the *N*-th value 216, and the baseline value of the fatigue parameter (step 112). This may allow the eye care practitioner to separate the visual fatigue level associated with the respective one of the first set 202, second set 204, and up to the *N*-th set 206 of optical parameters, from other fatigue types. For example, a calculated difference having a value lower than a predetermined threshold value may indicate that the first value 212, second value 214, and up to the *N*-th value 216 of the fatigue parameter may be attributed to the subject's visual fatigue level that is associated with the respective one of the first set 202, second set 204, and up to the *N*-th set 206 of optical parameters.

The final optical prescription 220 may be obtained based on the comparison of the determined values of the fatigue parameter (step 106), by selecting the determined value of the fatigue parameter corresponding to a lowest visual fatigue level, e.g. lowest determined value of the fatigue parameter, among the determined values of the fatigue parameter; and obtaining, as the final optical prescription 220, the respective one of the intermediate optical prescriptions associated with the determined value of the fatigue parameter corresponding to the lowest visual. For example, the final optical prescription 220 may be obtained by selecting the lowest value of the fatigue parameter, among the first value 212, the second value 214, and up to the *N*-th value 216 of the fatigue parameter, and selecting the corresponding set of optical parameters that corresponds to the lowest value of the fatigue parameter. Accordingly, the final optical prescription 230 thus corresponds to an optical prescription which provides a lower visual fatigue level and visual discomfort and good visual acuity.

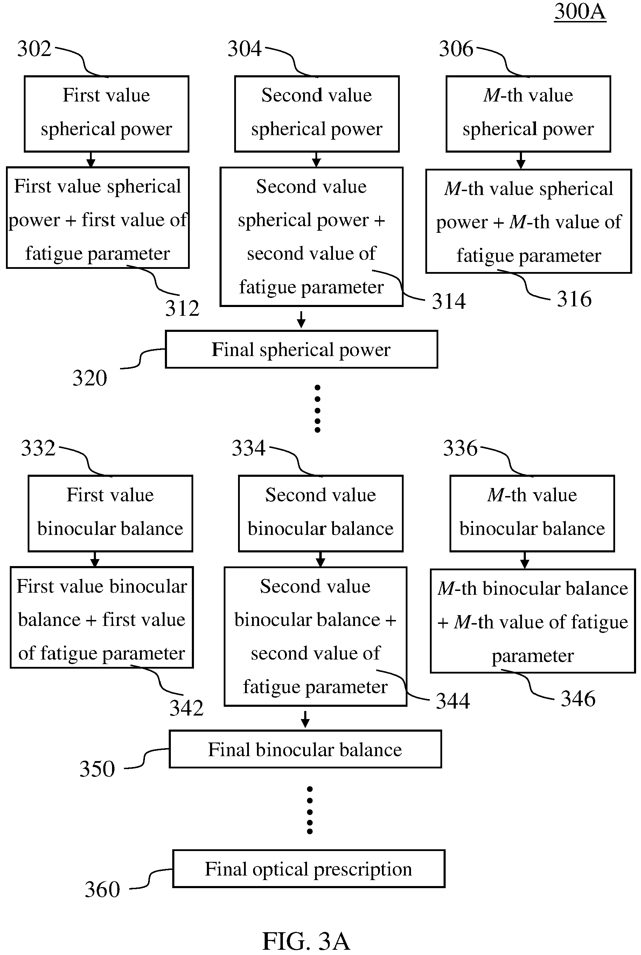

Figure 3A shows a schematic illustration of an exemplary embodiment 300A for obtaining the optical prescription, when the intermediate optical prescription comprises a value of a dioptric optical parameter. The dioptric optical parameter may be associated with a visual acuity condition of the subject. For example, the value of a dioptric optical parameter may correspond to a value for a subject's spherical parameter, a value for the subject's cylinder parameter, a value for the subject's cylinder axis parameter, a value for the subject's binocular balance parameter.

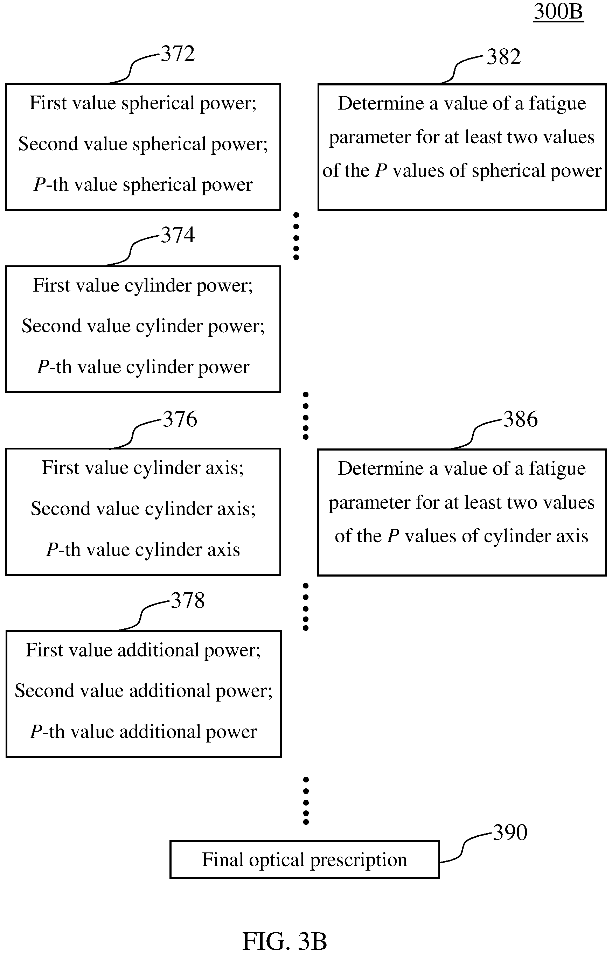

Referring to figures 1A, 1B, 3A and 3B, the values of the dioptric optical parameters, e.g. intermediate optical prescriptions, may be determined during the refraction examination (step 108), and/or may be received from an external source (step 110). In the example as shown in figure 3A, the first value 302, the second value 304, and up to theM-th, *M* ≥ 2, value 306, of the subject's spherical power may be obtained. The first value 302, second value 304, and up to theM-th value 306 of spherical power may each achieve the same or similar visual acuity for the subject.

Step 102 may thus include determining the value of the fatigue parameter, e.g. fatigue measurements, for at least two values of the first to M-th values 302, 304, 306 of the dioptric optical parameter, e.g. spherical power. As shown in figure 3A, a first value of the fatigue parameter 312 for the first value of the spherical power 302, a second value of the fatigue parameter 314 for the second value of spherical power 304, and up to theM-th, *M* ≥ 2, value of the fatigue parameter 316 for the M-th value of spherical power 306, may be obtained.

Step 104 may be performed to compare the determined values of the fatigue parameter. For example, the first value 312, the second value 314, up to the M-th value 316 of the fatigue parameter for spherical power may be compared against each other. In some embodiments, step 104 may further include calculating the difference between the determined values of the fatigue parameter, e.g. the first value 312, the second value 314, up to theM-th value 316, and the baseline value of the fatigue parameter (step 112), which may allow the eye care practitioner to separate the visual fatigue level associated with the respective one of the first value 302, second value 304, and up to theM-th value 306 of spherical power, with various other types of fatigue.

Step 106 may therefore include selecting the determined value of the fatigue parameter corresponding to a lowest visual fatigue level, e.g. lowest determined value of the fatigue parameter and obtaining, as the final spherical power 320, the respective one of the spherical powers associated with the determined value of the fatigue parameter corresponding to the lowest visual fatigue level. For example, the final spherical power 320 prescription may be obtained by selecting the lowest value of the fatigue parameter, among the first value 312, the second value 314, and up to the M-th value 316 of the fatigue parameter, and selecting the corresponding spherical power that corresponds to the lowest value of the fatigue parameter. The final spherical power 230 may be part of the final optical prescription 360 that minimizes visual fatigue and at the same time, provides good visual acuity for the subject.

In the embodiment as shown in figure 3A, steps 102, 104 and 106 may be repeated for each dioptric optical parameter of the intermediate optical prescription. For example, after obtaining the final spherical power 320, step 102 may be performed to determine the value of the fatigue parameter for at least two values of the dioptric optical parameter corresponding to the binocular balance of the subject. For example, a first value of the fatigue parameter 342 for a first value 332 of the binocular balance, a second value 344 of the fatigue parameter for a second value 334 of binocular balance, and up to an M-th value 346 of the fatigue parameter for an M-th value 336 of binocular balance, may be obtained. Step 104 may then be performed to compare the determined values of the fatigue parameter, e.g. the first value 342, the second value 344, and up to theM-th value 346 of the fatigue parameter for binocular balance. In some embodiments, step 104 may further include calculating the difference between the determined values of the fatigue parameter, and the baseline value of the fatigue parameter (step 112).

Step 106 may thus include selecting the determined value of the fatigue parameter corresponding to a lowest visual fatigue level, e.g. lowest determined value of the fatigue parameter and obtaining, as the final binocular balance 350, the respective one of the binocular balance values associated with the determined value of the fatigue parameter corresponding to the lowest visual fatigue level. The final binocular balance value 350 may be part of the final optical prescription 360 that minimizes visual fatigue and at the same time, provides good visual acuity for the subject.

In some embodiments, determination of the values of the fatigue parameter (step 102) may be performed continuously as the dioptric optical parameters are being determined (step 108) during the refraction examination and/or received (step 110) during the visual fatigue examination process. For example, each fatigue measurement may be associated with a timestamp during said examination process, and the measurements may be retrieved at the end of the examination. The visual fatigue measurements may therefore guide the refraction examination, or optimize the visual fatigue examination in real-time, such that a final optical prescription 360 which achieves good visual acuity and minimal visual fatigue may be obtained.

Figure 3B shows a schematic illustration of an exemplary embodiment 300B for obtaining the optical prescription, when the intermediate optical prescription comprises a value of a dioptric optical parameter. The dioptric optical parameter may be associated with a visual acuity condition of the subject. Figure 3B may be based on the embodiment described with reference to figure 3A and repeated descriptions will be omitted for brevity. However, in embodiment 300B, determination of the value of the fatigue parameter may be performed for selected dioptric optical parameters.

In embodiment 300B, the plurality of intermediate optical prescriptions, e.g. first to *P-*th, *P* ≥ 2, values of spherical power 372 may be determined (step 108) and/or received (step 110) as described above. Step 102 may be performed to determine a value of a fatigue parameter for at least two values of the P values of spherical power 382. In some embodiments, steps 104 and 106 may be performed to obtain the final spherical power.

Embodiment 300B may further include determining (step 108) and/or receiving (step 110) the plurality of intermediate optical prescriptions, e.g. first toP-th values of cylinder power 374. As shown in figure 3B, fatigue measurements may not be performed for said P values of cylinder power 374.

Embodiment 300B may further include determining (step 108) and/or receiving (step 110) the plurality of intermediate optical prescriptions, e.g. first to P-th values of cylinder axis 376. As shown in figure 3B, step 102 may be performed to determine a value of a fatigue parameter for at least two values among the P values of cylinder axis 386. In some embodiments, steps 108 and 110 may also be performed to obtain the final value for the cylinder axis.

Embodiment 300B may further include determining (step 108) and/or receiving (step 110) the plurality of intermediate optical prescriptions, e.g. first to P-th values of additional power 378. As shown in figure 3B, fatigue measurements may not be performed for said P values of additional power 378.

In embodiment 300B, the final optical prescription 390 may therefore be obtained based on the determined (step 108) and/or received (step 110) values of the dioptric optical parameter, and for selected dioptric optical parameters, determining the value of the fatigue parameter for said values of the dioptric optical parameter. In other words, fatigue measurements may be performed only for selected dioptric optical parameters, e.g. at selected time-points of the refraction or visual fatigue examination process. This may be helpful to expedite the examination process and prevent over-fatigue in the subject during said process. Furthermore, the fatigue measurements for the selected dioptric optical parameters may guide the determination of the final optical prescription 390. As such, an optical prescription 390 having good visual acuity and minimal visual fatigue may be obtained.

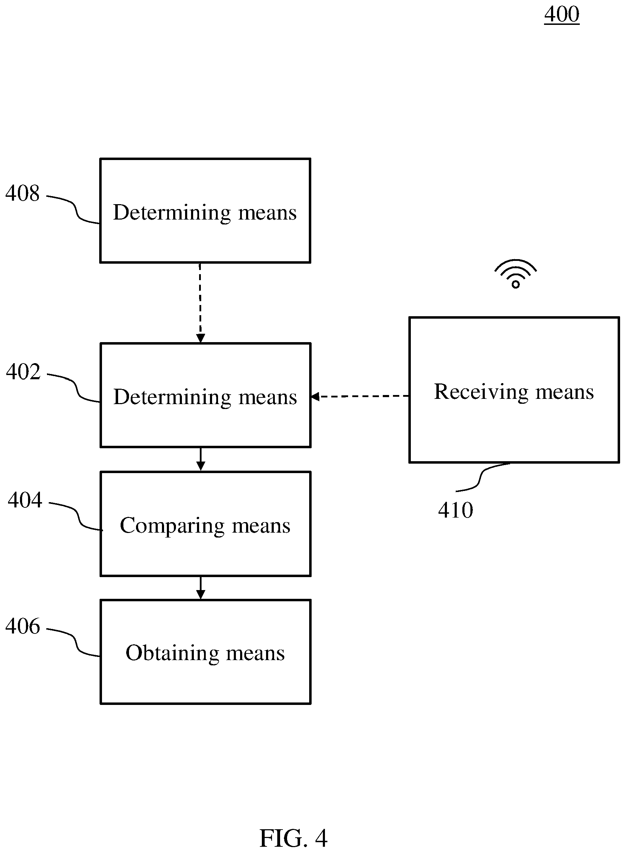

According to a second aspect of the disclosure, there is provided a system for obtaining an optical prescription. Figure 4 shows a schematic illustration of a system 400 for obtaining an optical prescription, in accordance with various embodiments. System 400 may be based on and configured to execute the steps of methods 100A, 100B, 200, 300A, 300B and repeated descriptions will be omitted for brevity.

System 400 includes (i.) means for determining a value of a fatigue parameter for at least two intermediate optical prescriptions among a plurality of intermediate optical prescriptions 402, the fatigue parameter associated with a visual fatigue level of a subject carrying out a visual task involving any kind of visual content, wherein each determined value of the fatigue parameter is associated with a respective one of the plurality of intermediate optical prescriptions; (ii.) means for comparing the determined values of the fatigue parameter 404; and (iii.) means for obtaining the optical prescription, based on the comparison of the determined values of the fatigue parameter 406.

In various embodiments, at least one of the means for determining the value of the fatigue parameter for the at least two intermediate optical prescriptions among the plurality of intermediate optical prescriptions 402, the means for comparing the determined values of the fatigue parameter 404, and the means for obtaining the optical prescription 406, comprises a circuit. A circuit may include analog circuits or components, digital circuits or components, or hybrid circuits or components. Any other kind of implementation of the respective functions which will be described in more detail below may also be understood as a "circuit" in accordance with an alternative embodiment. A digital circuit may be understood as any kind of a logic implementing entity, which may be special purpose circuitry or a processor executing software stored in a memory, firmware, or any combination thereof. Thus, in various embodiments, a "circuit" may be a digital circuit, e.g. a hard-wired logic circuit or a programmable logic circuit such as a programmable processor, e.g. a microprocessor (e.g. a Complex Instruction Set Computer (CISC) processor or a Reduced Instruction Set Computer (RISC) processor). A "circuit" may also include a processor executing software, e.g. any kind of computer program, e.g. a computer program using a virtual machine code such as e.g. Java. For example, at least one of the means for determining the value of the fatigue parameter for the at least two intermediate optical prescriptions among the plurality of intermediate optical prescriptions 402, the means for comparing the determined values of the fatigue parameter 404, and the means for obtaining the optical prescription 406, may be a microprocessor such as a fatigue testing instrument, e.g. eye-tracker, camera, and in some embodiments, a refraction testing instrument, e.g. refractors, optometers.

In some embodiments, as shown in figure 4, system 400 may further include means for determining the plurality of intermediate optical prescriptions 408, each intermediate optical prescription being different to another intermediate optical prescription among the plurality of optical prescription. The means for determining the plurality of intermediate optical prescriptions 408 may be part of, or may be included in the circuit, e.g. in the same microprocessor. For example, the fatigue testing instrument may be integrated with the refraction testing equipment. As such, the fatigue measurements may be performed during refraction examination, and may provide a direct and simple way of obtaining the optical prescription for the subject. In addition, the eye care practitioner does not need to perform additional fatigue measurements during the refraction examination process to obtain an optical prescription which provides good visual acuity and minimizes visual fatigue.

In some other embodiments, as shown in figure 4, the means for receiving the plurality of intermediate optical prescriptions 410, each intermediate optical prescription being different to another intermediate optical prescription among the plurality of intermediate optical prescriptions, may comprise the circuit. In other words, the plurality of intermediate optical prescription may be determined in a separate circuit, e.g. refraction testing instrument, and may be transmitted to the circuit comprising the means for determining the value of the fatigue parameter for the at least two intermediate optical prescriptions among the plurality of intermediate optical prescriptions 402, the means for comparing the determined values of the fatigue parameter 404, and the means for obtaining the optical prescription 406. For example, said circuit may be a standalone device, e.g. independent fatigue testing instrument. The circuit may therefore be equipped with the means for receiving the plurality of intermediate optical prescriptions 410. In this embodiment, the plurality of intermediate optical prescription, may be received according to a predefined wireless communication protocol. Examples of the predefined wireless communication protocols include: global system for mobile communication (GSM), enhanced data GSM environment (EDGE), wideband code division multiple access (WCDMA), code division multiple access (CDMA), time division multiple access (TDMA), wireless fidelity (Wi-Fi), voice over Internet protocol (VoIP), worldwide interoperability for microwave access (Wi-MAX), Wi-Fi direct (WFD), an ultra-wideband (UWB), infrared data association (IrDA), Bluetooth, ZigBee, SigFox, LPWan, LoRaWan, GPRS, 3G, 4G, LTE, and 5G communication systems. Accordingly, the means for receiving the plurality of intermediate optical prescriptions 410 may include the necessary hardware required to support the wireless transmission. Alternatively, the plurality of intermediate optical prescription may be received via wired means.

Advantageously, the various means for determining the value of the fatigue parameter 402, e.g. by performing at least one objective and/or subjective measurement, obtaining the at least one parameter relating to the subject may be easily combined in the circuit. In addition, means for displaying the visual content associated with the visual task may be integrated into the circuit.

According to another aspect of the disclosure, a computer program product may include instructions to cause the system 400 to execute the steps of method 100A, 100B, 200, 300A, 300B, for obtaining the optical prescription.

The present disclosure thus discloses methods and system for obtaining a subject-specific optical prescription that may be implemented in an optical article, e.g. eyewear, contact lenses. The methods and system may be used to guide, optimize and fine-tune the intermediate optical prescriptions by further determining the visual fatigue levels with respect to an intermediate optical prescription, to obtain an optical prescription. Said optical prescription provides good visual acuity, and at the same time, minimizes the visual fatigue level for the subject. This may improve the overall vision health for the subject, in particular, when the subject is engaging in distance, e.g. far- and near-work activities.

While the disclosure has been particularly shown and described with reference to specific embodiments, it should be understood by those skilled in the art that various changes in form and detail may be made therein without departing from the spirit and scope of the disclosure as defined by the appended claims. The scope of the disclosure is thus indicated by the appended claims and all changes which come within the meaning and range of equivalency of the claims are therefore intended to be embraced.

## Claims

1. A method (100A, 100B) for obtaining an optical prescription comprising
(i.) determining a value of a fatigue parameter for at least two intermediate optical prescriptions among a plurality of intermediate optical prescriptions, the fatigue parameter associated with a visual fatigue level of a subject carrying out a visual task involving any kind of visual content, wherein each determined value of the fatigue parameter is associated with a respective one of the plurality of intermediate optical prescriptions (102);
(ii.) comparing the determined values of the fatigue parameter (104);
(iii.) obtaining the optical prescription, based on the comparison of the determined values of the fatigue parameter (106).

2. The method (100A, 100B) of claim 1, further comprising
(i.) determining the plurality of intermediate optical prescriptions, each intermediate optical prescription being different to another intermediate optical prescription among the plurality of intermediate optical prescriptions (108).

3. The method (100A, 100B) of claim 1, further comprising
(i.) receiving the plurality of intermediate optical prescriptions, each intermediate optical prescription being different to another intermediate optical prescription among the plurality of intermediate optical prescriptions (110).

4. The method (100A, 100B) of any one of claims 1 to 3, wherein obtaining the optical prescription based on the comparison of the determined values of the fatigue parameter (106) comprises
(i.) selecting the determined value of the fatigue parameter corresponding to a lowest visual fatigue level, among the determined values of the fatigue parameter;
(ii.) obtaining the optical prescription, based on the respective one of the intermediate optical prescriptions associated with the determined value of the fatigue parameter corresponding to the lowest visual fatigue level.

5. The method (100A, 100B, 300A, 300B) of any one of claims 1 to 4, wherein each of the plurality of intermediate optical prescriptions comprises a value of a dioptric optical parameter, the dioptric optical parameter associated with a visual acuity condition of the subject.

6. The method (100A, 100B, 200) of any one of claims 1 to 4, wherein each of the plurality of intermediate optical prescriptions comprises a set of optical parameters, the set of optical parameters comprising a plurality of dioptric optical parameters and a corresponding value associated with a respective one of the plurality of dioptric optical parameters, wherein the plurality of dioptric parameters are associated with a visual acuity condition of the subject.

7. The method (100A, 100B, 200, 300A, 300B) of any one of claims 1 to 6, wherein determining the value of the fatigue parameter for each intermediate optical prescription among the plurality of intermediate optical prescriptions comprises
(i.) performing at least one objective measurement relating to the subject;
(ii.) performing at least one subjective measurement relating to the subject;
(iii.) obtaining at least one parameter relating to information about the subject,
or any combination of the above.

8. The method (100A, 100B, 200, 300A, 300B) of claim 7, wherein the at least one objective measurement relating to the subject comprises: at least one pupil-tracking measurement, a critical flicker fusion frequency measurement, an accommodation micro-fluctuation measurement, a blink frequency measurement, a blink amplitude measurement, and/or a measurement relating to the subject's facial features, related to the visual fatigue level of the subject carrying out the visual task involving any kind of visual content.

9. The method (100A, 100B, 200, 300A, 300B) of claim 7 or claim 8, wherein the at least one subjective measurement relating to the subject comprises at least one answer by the subject to at least one question, related to the visual fatigue level of the subject carrying out the visual task involving any kind of visual content.

10. The method (100A, 100B, 200, 300A, 300B) of any one of claims 1 to 9, further comprising
(i.) determining a baseline value of the fatigue parameter, the baseline value associated with a baseline visual fatigue level of the subject carrying out the visual task involving any kind of visual content (112), wherein the baseline value of the fatigue parameter is determined before determining the plurality of intermediate optical prescriptions.

11. The method (100A, 100B, 200, 300A, 300B) of claim 10, further comprising
(i.) calculating a difference between the determined value of the fatigue parameter associated with the respective one of the plurality of intermediate optical prescription, and the baseline value of the fatigue parameter.

12. A system (400) for obtaining an optical prescription comprising
(i.) means for determining a value of a fatigue parameter for at least two intermediate optical prescriptions among a plurality of intermediate optical prescriptions (402), the fatigue parameter associated with a visual fatigue level of a subject carrying out a visual task involving any kind of visual content, wherein each determined value of the fatigue parameter is associated with a respective one of the plurality of intermediate optical prescriptions;
(ii.) means for comparing the determined values of the fatigue parameter (404);
(iii.) means for obtaining the optical prescription, based on the comparison of the determined values of the fatigue parameter (406).

13. The system (400) of claim 12, wherein at least one of the means for determining the value of the fatigue parameter for the at least two intermediate optical prescriptions among the plurality of intermediate optical prescriptions (402), the means for comparing the determined values of the fatigue parameter (404), the means for obtaining the optical prescription (406), comprises a circuit.

14. The system (400) of claim 13, further comprising
(i.) means for determining the plurality of intermediate optical prescriptions (408), each intermediate optical prescription being different to another intermediate optical prescription among the plurality of intermediate optical prescriptions, wherein the means for determining the plurality of intermediate optical prescriptions comprises the circuit;
(ii.) means for receiving the plurality of intermediate optical prescriptions (410), each intermediate optical prescription being different to another intermediate optical prescription among the plurality of intermediate optical prescriptions, wherein the means for receiving the plurality of intermediate optical prescriptions comprises the circuit,
or any combination of the above.

15. A computer program product, comprising instructions to cause the system (400) of any one of claims 12 to 14 to execute the steps of the method (100A, 100B, 200, 300A, 300B) of any one of claims 1 to 11.
